# EUROPEAN PATENT SPECIFICATION

(11) **EP 4 112 022 B1**
(45) Date of publication and mention of the grant of the patent: **11.09.2024**
(21) Application number: 21182498.2
(22) Date of filing: 29.06.2021
(51) Int. Cl.: A61F 13/15, A61F 13/494, A61F 13/495

(54) **AN ABSORBENT SANITARY ARTICLE AND A METHOD FOR PRODUCING THE SAME**
ABSORBIERENDER HYGIENEARTIKEL UND VERFAHREN ZUR HERSTELLUNG DAVON
ARTICLE SANITAIRE ABSORBANT ET SON PROCÉDÉ DE PRODUCTION

(43) Date of publication of application: 04.01.2023
(73) Proprietor: Fameccanica.Data S.p.A., 66020 San Giovanni Teatino (CH) (IT)
(72) Inventor: SABLONE, Gabriele, 66020 San Giovanni Teatino (Chieti) (IT)
(74) Representative: Marchitelli, Mauro

(56) References cited:
- EP-A1- 3 406 234
- WO-A1-2018/143921
- US-A1- 2019 290 509

## Description

### Field of the invention

The present invention relates to absorbent sanitary articles, such as, for example, diapers, training pants, absorbent sanitary products for incontinent adults, etc.

The present invention also relates to a method for producing absorbent sanitary articles.

### Prior art

An absorbent sanitary article typically has a structure that comprises a central body or chassis having a front section and a rear section. The chassis normally includes a permeable topsheet intended to come into contact with the user's skin when the article is worn, an impermeable backsheet and an absorbent core sandwiched between the topsheet and the backsheet. The front and rear sections of the chassis are normally closed around the user's waist by means of hook-and-loop fasteners, better known as Velcro^{©} fastening devices.

The front section and rear section may be elasticized. WO 2020/242714 and WO2021/092606 disclose methods for bonding elastic parts under tension to an advancing carrier, which may be used for elasticizing the waist sections of absorbent sanitary articles. The methods disclosed in these documents provide: advancing a carrier substrate, advancing a continuous elastic substrate, cutting an elastic part from the continuous elastic substrate, stretching the elastic part in a cross direction, positioning the elastic part on the carrier substrate, and adhesively bonding a stretched central region of the elastic part with the carrier substrate, and mechanically bonding end regions of the elastic part with the carrier substrate. EP3406234 discloses an absorbent sanitary article; WO2018/143921 and US2019/290509 represent relevant prior art in the technical field.

The formation of elasticized waist sections involves a high cost both for the machine and for the materials used for manufacturing absorbent sanitary articles.

Although it would be desirable to avoid applying elastic elements in the waist regions to reduce the costs of absorbent sanitary articles and of the machines for manufacturing the same, without elastic elements in the waist regions the absorbent sanitary articles might have reduced capacity to contain leakage of body exudates in the crotch region, especially solid and semi-solid body exudates.

### Object and summary of the invention

The object of the present invention is to provide an absorbent sanitary article which overcomes the problems of the prior art.

More specifically the object of the present invention is to provide low cost absorbent sanitary articles which maintain good capacity of retention of body exudates.

According to the invention, this object is achieved by an absorbent sanitary article having the features of claim 1.

According to another aspect, the invention relates to a method for manufacturing absorbent sanitary articles having the features of claim 9.

The claims form an integral part of the technical disclosure provided here in relation to the invention.

### Brief description of the drawings

The present invention will now be described with reference to the attached drawings, provided purely by way of non-limiting example, wherein:
- Figure 1 is a perspective view of an absorbent sanitary article according to the present invention,
- Figure 2 is a schematic plan view showing the absorbent sanitary article of figure 1 in an extended position,
- Figure 3 is a schematic plan view showing another embodiment of an absorbent sanitary article according to the invention in an extended position, and
- Figure 4 is a schematic view of an embodiment of a machine for manufacturing absorbent sanitary articles according to the present invention.

It should be appreciated that the attached drawings are schematic and not to scale with respect to real products. Various figures may not be represented in the same scale. Also, in various figures some elements may not be shown to better show other elements.

### Detailed description

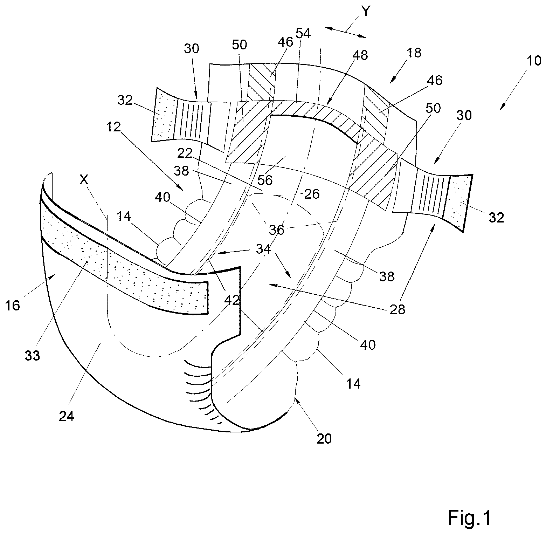

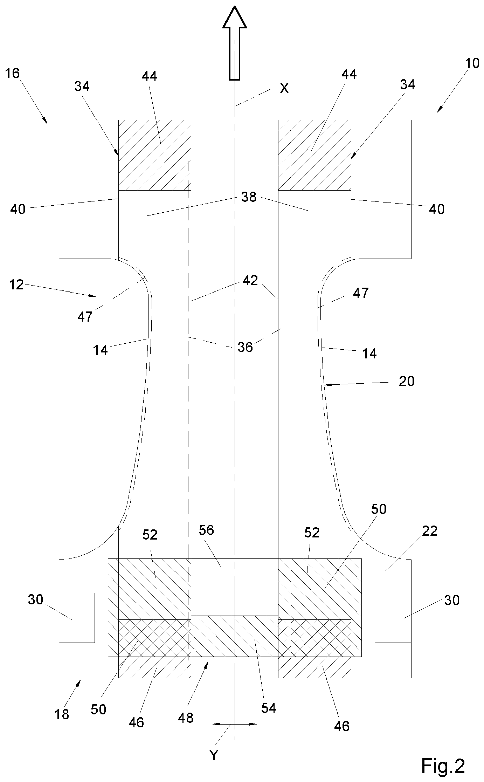

With reference to Figures 1 and 2, numeral 10 indicates an absorbent sanitary article according to the present invention. Figure 1 shows the absorbent sanitary article 10 in the configuration in which it is worn, and Figure 2 shows the absorbent sanitary article 10 in a flat configuration.

The absorbent sanitary article 10 comprises a chassis 12 elongated along a longitudinal axis X. The chassis 12 has two side edges 14, a front section 16 and a rear section 18. The front section 16 and the rear section 18 in use are closed around the user's waist. The front section 16 and the rear section 18 may be not elastic. A crotch section 20 extends between the front section 16 and the rear section 18. In use, the crotch section 20 is arranged between the legs of the user.

The side edges 14 may be cut so that they conform to the legs of the user in the configuration in which the absorbent sanitary article 10 is worn. The side edges 14 may be shaped so that in a flat configuration the absorbent sanitary article 10 has substantially an hourglass shape.

The chassis 12 comprises a topsheet 22 made of a permeable material having an outer surface which, in use, is in contact with the user's skin, an impermeable backsheet 24, and an absorbent core 26 sandwiched between the topsheet 22 and the backsheet 24.

The absorbent sanitary article 10 comprises a fastening device 28 configured for fastening the front section 16 and the rear section 18 to each other. The fastening device 28 may comprise two back side panels 30 attached to the rear section 18 of the chassis 12 and extending laterally beyond respective side edges 14. The back side panels 30 may be elastically stretchable in a transverse direction Y orthogonal to the longitudinal axis X. The back side panels 30 may have distal portions provided with micro-hook elements 32 which form a releasable surface connection with a micro-loop front panel 33 attached in the front section 16 of the chassis 12.

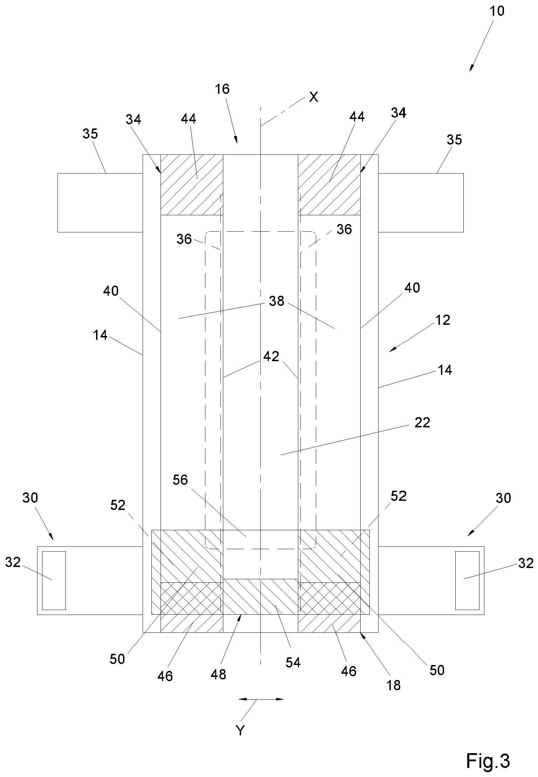

With reference to figure 3, in a possible embodiment the chassis 12 may have a rectangular shape, with two straight side edges 14 parallel to the longitudinal axis X. The absorbent sanitary article 10 may comprises a pair of front side panels 35 projecting laterally from opposite sides of the front section 16. The front side panels 35 may have a surface of loop material (e.g. a non-woven material) for connection with the micro-hook elements 32 of the back side panels 30. In this case the micro-loop front panel 33 may not be present.

Both in the embodiment of figures 1, 2 and in the embodiment of figure 3, at least one of the transverse edges of the chassis 12 may be shaped instead of straight. For instance, the front edge may have a central substantially semi-circular concave portion to contour the umbilical cord of the user.

The absorbent sanitary article 10 comprises two elastic leg cuffs 34. The leg cuffs 34 are sometimes also referred to as leg bands, side flaps, barrier cuffs, elastic cuffs or gasketing cuffs. The elastic leg cuffs 34 may be formed as disclosed in US3860003, US4909803, US4695278, US4795454, US4704115, and US2009/0312730 A1.

The elastic leg cuffs 34 comprise a plurality of elastic wires 36 which are fixed, e.g. by glue, at discrete points to two opposite non-woven layers 38.

The non-woven layers 38 of the elastic leg cuffs 34 have respective outwardly facing longitudinal edges 40 which are fixed, e.g. by glue, to the outer surface of the topsheet 22. In the crotch section 20 the outwardly facing longitudinal edges 40 of the elastic leg cuffs 34 may be adjacent to the respective side edges 14 of the chassis 12.

The non-woven layers 38 of the elastic leg cuffs 34 have respective inwardly facing longitudinal edges 42 detached from the outer surface of the topsheet 22 which in use are in contact with the legs of the user to form barriers which help reduce the leakage of liquid, solid or semi-solid body exudates in the leg regions. At least some of the elastic wires 36 may extend along the inwardly facing longitudinal edges 42 of the elastic leg cuffs 34.

The elastic leg cuffs 34 have respective non-elastic end portions 44, 46. At the non-elastic end portions 44, 46 the outwardly facing longitudinal edges 40 and the inwardly facing longitudinal edges 42 are both fixed to the outer surface of the topsheet 22. The non-elastic end portions 44, 46 may be rendered non-elastic by not providing the elastic wires 36 at these portions or by not fixing the elastic wires 36 to the non-woven layers 38 at these portions.

The elastic leg cuffs 34 may be configured in various ways. In a possible embodiment the elastic leg cuffs 34 may be formed separately from the topsheet 22 by sandwiching longitudinally stretched elastic wires 36 between two non-woven layers 38. The longitudinally tensioned elastic wires 36 are fixed in discrete points to the two non-woven layers 38 by glue or by welds forming mechanical anchoring points, as disclosed in EP3092997 and US6291039. Between the longitudinally spaced fixing points the elastic wires 36 are free to contract, so that the two non-woven layers 38 assume a pleated shape when the elastic tension of the elastic wires 36 is released.

It should be appreciated that the elastic leg cuffs 34 may be formed in various ways, such as for example, by folding portions of the topsheet 22 laterally inward, i.e., toward the longitudinal axis X, to form both the leg cuffs 34 and the side edges 14 of the chassis 12. In another example, the leg cuffs 24 may be formed by attaching an additional non-woven layer or layers to the chassis 12 at or adjacent to each of the respective side edges 14 of the chassis 12. The leg cuffs 34 may extend to the same longitudinal extent as the chassis 12 or alternatively the leg cuffs 34 may have a longitudinal extent that is less than that of the chassis 12.

The absorbent sanitary article 10 may comprise leg elastic elements 47 which may be formed by stretched elastic wires fixed between the topsheet 22 and the backsheet 24 and extending along the side edges 14 of the chassis 12, which impart a pleated form to the side edges 14 in the crotch section 20.

The absorbent sanitary article 10 comprises at least one gasketing element 48 elongated in a transverse direction Y orthogonal to the longitudinal axis X.

The at least one gasketing element 48 is attached to the outer surface of the topsheet 22 in at least one of the front and rear waist sections 16, 18. In a possible embodiment the absorbent sanitary article 10 may comprise two gasketing elements 48 attached to the outer surface of the topsheet 22 in the front waist section 16 and in the rear waist section 18, respectively.

The or each gasketing element 48 is not stretched in the transverse direction Y and has a length in the transvers direction Y which is less than the length in the transverse direction of the chassis 12. This simplifies and reduces the cost of the machine for manufacturing the absorbent sanitary articles in that a device for stretching transversally the gasketing elements 48 is not required.

The or each gasketing element 48 has two opposite lateral portions 50 overlapped and fixed to elastic end portions 52 of the respective elastic leg cuffs 34. The two opposite lateral portions 50 of the gasketing element 48 extend partially on the non-elastic end portions 44, 46 and partially on the elastic end portions 52 of the elastic leg cuffs 34. The two opposite lateral portions 50 of the gasketing element 48 may be fixed to the corresponding portions 44, 46, 52, of the leg cuffs 34 by glue or by welding, e.g. ultrasonic welding or thermocompression welding.

The or each gasketing element 48 has a central outer portion 54 extending between the two opposite lateral portions 50 and fixed to the outer surface of the topsheet 22, e.g. by glue, ultrasonic welding or thermocompression welding.

The or each gasketing element 48 has a central inner portion 56 extending between the two opposite lateral portions 50 and detached from the outer surface of the topsheet 22.

When the absorbent sanitary article is worn, the or each gasketing element 48 is in contact with the back and or front waist region of the user. The elastic leg cuffs 34 draw elastically inwardly the central inner portion 56 of the or each gasketing element 48. The central inner portion 56 forms an inwardly open pocket between the outer surface of the topsheet and the or each gasketing element 48. Such pocket helps containing liquid, solid or semi-solid body exudates in the back and/or front waist region.

In the preferred embodiment, said at least one gasketing element 48 is made of a non-elastic material. At least one gasketing element 48 may comprise a layer of impermeable plastic material forming an impermeable barrier in the back and/or front waist region which helps containing body exudates. At least one gasketing element 48 may comprise a layer of non-woven material on a surface opposite the outer surface of the topsheet 22 to provide a soft feeling against the skin of the user. In a possible embodiment at least one gasketing element 48 may be formed by a non-elastic laminate including a layer of impermeable plastic material and at least one layer of a non-woven material.

In a possible embodiment, at least one gasketing element 48 is made of an elastic material not stretched in the transverse direction Y. In a possible embodiment at least one gasketing element 48 may be formed by an elastic laminate including an elastic film and at least one layer of a non-woven material.

In a possible embodiment, at least one gasketing element 48 has a transverse outer edge aligned to a corresponding transverse edge of the respective front or rear waist section 16, 18.

Since the or each gasketing element 48 is not stretched in the transverse direction, it may have a surface with a printed pattern including writings, and/or images.

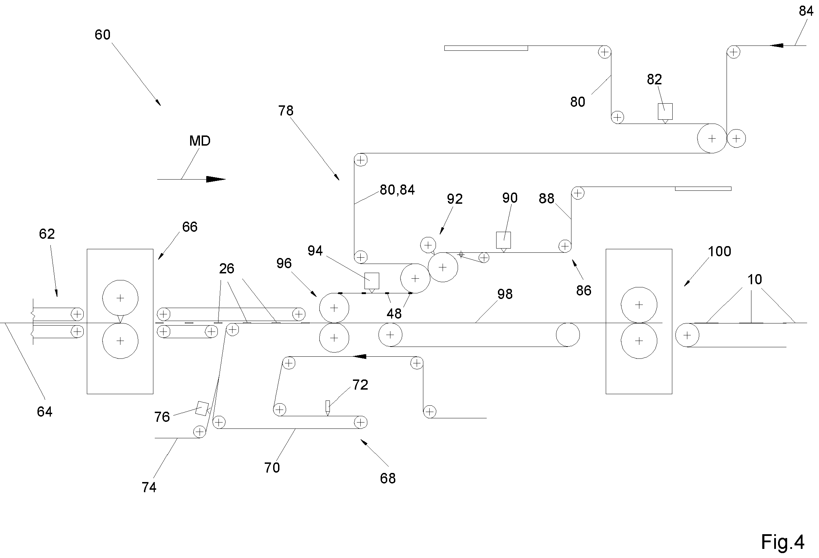

The absorbent sanitary articles 10 according to the present invention may be manufactured by a machine 60 schematically shown in figure 4.

The machine 60 comprises an inlet conveyor 62 which feeds in a machine direction MD a continuous absorbent tape 64. A first cutting unit 66 transversally cuts the continuous absorbent tape 64 to form individual absorbent cores 26 moving in the machine direction MD.

A first web feeding device 68 feeds a continuous backsheet web 70 in the machine direction MD. A first glue dispenser 72 applies glue on an upper surface of the continuous backsheet web 70.

The individual absorbent cores 26 moving in the machine direction MD are applied on the upper surface of the continuous backsheet web 70.

Continuous elastic wires 74 intended to form leg elastic elements 47 are applied on the upper surface of the continuous backsheet web 70. A second glue dispenser 76 applies glue to the continuous elastic wires 74.

A second web feeding device 78 feeds a continuous topsheet web 80 in the machine direction MD. A third glue dispenser 82 applies glue on a first surface of the continuous topsheet web 80.

Two continuous elastic leg cuffs bands 84 are applied to the first surface of the continuous topsheet web 80, where they are retained by glue.

A third web feeding device 78 feeds a continuous gasketing web 88 in the machine direction MD. A fourth glue dispenser 90 applies glue on a surface of the continuous gasketing web 88.

A cut-and-slip unit 92 transversally cuts the continuous gasketing web 88 to form individual gasketing elements 48 elongated in a transverse direction and spaced apart from each other in a longitudinal direction. The individual gasketing elements 48 are applied on the first surface of the continuous topsheet web 80 not stretched in the transverse direction Y. The gasketing elements 48 are retained by glue on the first surface of the continuous topsheet web 80.

A fifth glue dispenser 94 applies glue on a second surface of the continuous topsheet web 80.

The continuous topsheet web 80 with the two continuous elastic leg cuffs bands 84 and the individual gasketing elements 48 applied to the first surface continuous topsheet web 80 is then overlapped to the continuous backsheet web 70, with the second surface of the continuous topsheet web 80 facing the upper surface of the continuous backsheet web 70.

A compression unit 96 compresses together the continuous topsheet web 80 and the continuous backsheet web 70 with the individual absorbent cores 26 sandwiched therebetween, to form a continuous chassis chain 98.

Pairs of side panels 30 may be attached to the continuous backsheet web 70, to the continuous backsheet web 70 or to the continuous chassis chain 98.

The continuous elastic leg cuffs bands 84 and the individual gasketing elements 48 spaced apart from each other in the machine direction MD may be applied to the first surface of the continuous topsheet web 80 after the continuous topsheet web 80 is overlapped to the continuous backsheet web 70.

A second cutting unit 100 cuts the continuous chassis chain 98 to form individual absorbent sanitary articles 10. The second cutting unit 100 may also cut the side edges of the continuous chassis chain 98 to form shaped side edges 14.

A method for producing absorbent sanitary articles according to the present invention comprises:
- providing a continuous topsheet web 80,
- attaching two continuous leg cuffs bands 84 to a first surface of said continuous topsheet web 82,
- providing a continuous gasketing web 88,
- cutting said continuous gasketing web 88 in a transverse direction so as to form individual gasketing elements 48 elongated in a transverse direction Y and spaced apart from each other in a longitudinal direction,
- after attaching the two continuous leg cuffs bands 84 to the first surface of the continuous topsheet web 80, overlapping and fixing opposite lateral portions 50 of the gasketing elements 48 not stretched in the transverse direction Y to elastic portions 52 of the two continuous leg cuffs bands 84, fixing central outer portions 54 of the gasketing elements 48 extending between respective opposite lateral portions 50 to the first surface of the continuous topsheet web 80 and leaving central inner portions 56 of the gasketing elements 48 extending between respective opposite lateral portions 50 detached from the first surface of the continuous topsheet web 80, and
- arranging an array of absorbent cores 26 between the continuous topsheet web 80 and a continuous backsheet web 70, so as to form a chassis chain 98,
- cutting said chassis chain 98 so as to form individual absorbent sanitary articles 10.

The step of attaching the two continuous leg cuffs bands 84 to the first surface of the continuous topsheet web 80 may be carried out before or after sandwiching the array of absorbent cores 26 between the continuous topsheet web 80 and the continuous backsheet web 70.

The method may comprise forming on the two continuous leg cuffs bands 84 non-elastic portions 44, 46 spaced apart from each other in a longitudinal direction, and overlapping and fixing the opposite lateral portions 50 of the gasketing elements 48 partially on the non-elastic portions 44, 46 and partially on elastic portions 52 of the continuous elastic leg cuffs bands 84.

Of course, without prejudice to the principle of the invention, the details of construction and the embodiments can be varied, even significantly, with respect to those illustrated here without departing from the scope of the invention as defined by the following claims.

## Claims

1. An absorbent sanitary article comprising:
- a chassis (12) having a longitudinal axis (X), a front and a rear waist section (16, 18), and a crotch section (20) intermediate between the front and rear waist section (16, 18), the chassis (12) including a topsheet (22) having an outer surface which in use is in contact with the user's skin, a backsheet (24), and an absorbent core (26) disposed between the topsheet (22) and the backsheet (24),
- a pair of elastic leg cuffs (34) extending at least in part along directions parallel to said longitudinal axis (X) and attached to said outer surface of the topsheet (22),
- at least one gasketing element (48) having a longitudinal axis oriented in a transverse direction (Y) orthogonal to said longitudinal axis (X), wherein the at least one gasketing element (48) has a length in the transverse direction (Y) which is less than the length in the transverse direction of the chassis (12), wherein the at least one gasketing element (48) is attached to said outer surface of the topsheet (22) in at least one of said front and a rear waist section (16, 18), wherein said at least one gasketing element (48) is not stretched in said transverse direction (Y) and has two opposite lateral portions (50) overlapped and fixed to elastic end portions (52) of said elastic leg cuffs (34), a central outer portion (54) extending between said two opposite lateral portions (50) and fixed to the outer surface of the topsheet (22) and a central inner portion (56) extending between said two opposite lateral portions (50) and detached from the outer surface of the topsheet (22), wherein said central inner portion (56) of the gasketing element (48) is raised from the outer surface of the topsheet (22) and forms a pocket open toward said crotch section (20),
**characterized in that** said at least one gasketing element (48) is made of a non-elastic material, and that said two opposite lateral portions (50) of the gasketing element (48) extend partially on non-elastic end portions (44, 46) and partially on elastic end portions (52) of the elastic leg cuffs (34).

2. The absorbent sanitary article of claim 1, wherein said at least one gasketing element (48) comprises an elastic material not stretched in said transverse direction (Y).

3. The absorbent sanitary article of any of the preceding claims, wherein said at least one gasketing element (48) comprises a layer of impermeable plastic material.

4. The absorbent sanitary article of claim 3, wherein said layer of impermeable plastic material forms the surface of the gasketing element (48) facing the outer surface of the topsheet (22).

5. The absorbent sanitary article of any of the preceding claims, wherein said at least one gasketing element comprises at least one layer of non-woven material.

6. The absorbent sanitary article of claim 5, wherein said at least one layer of non-woven material forms the surface of the gasketing element (48) which in use is in contact with the user's skin.

7. The absorbent sanitary article of any of the preceding claims, wherein said at least one gasketing element (48) has an outer edge aligned to a corresponding transverse edge of the respective front or rear waist section (16, 18).

8. The absorbent sanitary article of any of the preceding claims, wherein the chassis (12) has at least one transverse edge with a non-straight shaped profile.

9. A method for producing absorbent sanitary articles, comprising:
- providing a continuous topsheet web (80),
- attaching two continuous leg cuffs bands (84) to a first surface of said continuous topsheet web (80),
- providing a continuous gasketing web (88) made of a non-elastic material,
- cutting said continuous gasketing web (88) in a transverse direction so as to form individual gasketing elements (48) elongated in a transverse direction (Y) and spaced apart from each other in a longitudinal direction,
- after attaching the two continuous leg cuffs bands (84) to the first surface of the continuous topsheet web (80), overlapping and fixing opposite lateral portions (50) of the gasketing elements 48 not stretched in the transverse direction (Y) to elastic portions (52) of the two continuous leg cuffs bands (84), wherein said two opposite lateral portions (50) of the gasketing element (48) extend partially on non-elastic end portions (44, 46) and partially on elastic end portions (52) of the elastic leg cuffs (34), fixing central outer portions (54) of the gasketing elements (48) extending between respective opposite lateral portions (50) to the first surface of the continuous topsheet web (80) and leaving central inner portions (56) of the gasketing elements (48) extending between respective opposite lateral portions (50) detached from the first surface of the continuous topsheet web (80), and
- arranging an array of absorbent cores (26) between said continuous topsheet web (80) and continuous backsheet web (70), so as to form a chassis chain (98),
- cutting said chassis chain (98) so as to form individual absorbent sanitary articles (10).

10. The method of claim 9, wherein the step of attaching the two continuous leg cuffs bands (84) to the first surface of the continuous topsheet web (82) is carried out before sandwiching the array of absorbent cores (26) between the continuous topsheet web (80) and the continuous backsheet web (70).

11. The method of claim 10, wherein the step of attaching the two continuous leg cuffs bands (84) to the first surface of the continuous topsheet web (80) is carried out after sandwiching the array of absorbent cores (26) between the continuous topsheet web (80) and the continuous backsheet web (70).

12. The method of any of claims 9-11, comprising forming on the two continuous leg cuffs bands (84) non-elastic portions (44, 46) spaced apart from each other in a longitudinal direction, and overlapping and fixing said opposite lateral portions (50) of said gasketing elements (48) partially on said non-elastic portions (44, 46) and partially on elastic portions (52) of the continuous elastic leg cuffs bands (84).

## Patentansprüche

1. Absorbierender Hygieneartikel, umfassend:
- ein Rahmen (12), der eine Längsachse (X), einen vorderen und einen hinteren Taillenabschnitt (16, 18), und einen Schrittabschnitt (20) zwischen dem vorderen und dem hinteren Taillenabschnitt (16, 18) aufweist, wobei der Rahmen (12) ein oberes Blatt (22), das eine äußere Oberfläche aufweist, die bei Gebrauch mit der Haut des Benutzers in Kontakt steht, ein hinteres Blatt (24), und einen absorbierenden Kern (26) einschließt, der zwischen dem oberen Blatt (22) und dem hinteren Blatt (24) angeordnet ist,
- ein Paar elastischer Beinmanschetten (34), die sich zumindest teilweise entlang von Richtungen parallel zur Längsachse (X) erstrecken und an der äußeren Oberfläche des oberen Blattes (22) angebracht sind,
- mindestens ein Dichtungselement (48), das eine Längsachse aufweist, die in einer Querrichtung (Y) orthogonal zur Längsachse (X) ausgerichtet ist, wobei das mindestens eine Dichtungselement (48) eine Länge in der Querrichtung (Y) aufweist, die geringer ist als die Länge in der Querrichtung des Rahmens (12), wobei das mindestens eine Dichtungselement (48) an der äußeren Oberfläche des oberen Blattes (22) in mindestens einem des vorderen und des hinteren Taillenabschnitts (16, 18) angebracht ist, wobei das mindestens eine Dichtungselement (48) in der Querrichtung (Y) nicht gedehnt ist und zwei gegenüberliegende seitliche Abschnitte (50) aufweist, die überlappt sind und an elastischen Endabschnitten (52) der elastischen Beinmanschetten (34) befestigt sind, einen zentralen Außenabschnitt (54), der sich zwischen den beiden gegenüberliegenden seitlichen Abschnitten (50) erstreckt und an der äußeren Oberfläche des oberen Blattes (22) befestigt ist, und einen zentralen Innenabschnitt (56), der sich zwischen den beiden gegenüberliegenden seitlichen Abschnitten (50) erstreckt und von der äußeren Oberfläche des oberen Blattes (22) gelöst ist, wobei der zentrale Innenabschnitt (56) des Dichtungselements (48) von der äußeren Oberfläche des oberen Blattes (22) erhöht ist und eine zum Schrittabschnitt (20) hin offene Tasche bildet,
**dadurch gekennzeichnet, dass** das mindestens eine Dichtungselement (48) aus einem nicht elastischen Material hergestellt ist, und dass sich die beiden gegenüberliegenden seitlichen Abschnitte (50) des Dichtungselements (48) teilweise auf nicht elastischen Endabschnitten (44, 46) und teilweise auf elastischen Endabschnitten (52) der elastischen Beinmanschetten (34) erstrecken.

2. Absorbierender Hygieneartikel nach Anspruch 1, wobei das mindestens eine Dichtungselement (48) ein elastisches Material umfasst, das in der Querrichtung (Y) nicht gedehnt ist.

3. Absorbierender Hygieneartikel nach einem der vorstehenden Ansprüche, wobei das mindestens eine Dichtungselement (48) eine Schicht aus undurchlässigem Kunststoffmaterial umfasst.

4. Absorbierender Hygieneartikel nach Anspruch 3, wobei die Schicht aus undurchlässigem Kunststoffmaterial die Oberfläche des Dichtungselements (48) bildet, die der äußeren Oberfläche des oberen Blattes (22) zugewandt ist.

5. Absorbierender Hygieneartikel nach einem der vorstehenden Ansprüche, wobei das mindestens eine Dichtungselement mindestens eine Schicht aus Vliesmaterial umfasst.

6. Absorbierender Hygieneartikel nach Anspruch 5, wobei die mindestens eine Schicht aus Vliesmaterial die Oberfläche des Dichtungselements (48) bildet, die im Gebrauch mit der Haut des Benutzers in Kontakt steht.

7. Absorbierender Hygieneartikel nach einem der vorstehenden Ansprüche, wobei das mindestens eine Dichtungselement (48) eine Außenkante aufweist, die mit einer entsprechenden Querkante des jeweiligen vorderen oder hinteren Taillenabschnitts (16, 18) ausgerichtet ist.

8. Absorbierender Hygieneartikel nach einem der vorstehenden Ansprüche, wobei der Rahmen (12) mindestens eine Querkante mit einem nicht geradlinig geformten Profil aufweist.

9. Verfahren zur Herstellung eines absorbierenden Hygieneartikels, umfassend:
- Bereitstellen einer kontinuierlichen oberen Blattbahn (80),
- Anbringen von zwei kontinuierlichen Beinmanschettenbünden (84) an einer ersten Oberfläche der kontinuierlichen oberen Blattbahn (80),
- Bereitstellen einer kontinuierlichen Dichtungsbahn (88), hergestellt aus einem nicht elastischen Material,
- Schneiden der kontinuierlichen Dichtungsbahn (88) in einer Querrichtung, um einzelne Dichtungselemente (48) zu bilden, die in einer Querrichtung (Y) verlängert und in einer Längsrichtung voneinander beabstandet sind,
- nach Anbringen der beiden kontinuierlichen Beinmanschettenbünde (84) an der ersten Oberfläche der kontinuierlichen oberen Blattbbahn (80), Überlappen und Befestigen gegenüberliegender seitlicher Abschnitte (50) der Dichtungselemente 48, die in der Querrichtung (Y) nicht gedehnt sind, an elastischen Abschnitten (52) der beiden kontinuierlichen Beinmanschettenbünde (84), wobei sich die beiden gegenüberliegenden seitlichen Abschnitte (50) des Dichtungselements (48) teilweise auf nicht elastischen Endabschnitten (44, 46) und teilweise auf elastischen Endabschnitten (52) der elastischen Beinmanschetten (34) erstrecken, Befestigen von zentralen Außenabschnitten (54) der Dichtungselemente (48), die sich zwischen jeweiligen gegenüberliegenden seitlichen Abschnitten (50) erstrecken, an der ersten Oberfläche der kontinuierlichen oberen Blattbahn (80), und gelöst Belassen von zentralen Innenabschnitten (56) der Dichtungselemente (48), die sich zwischen jeweiligen gegenüberliegenden seitlichen Abschnitten (50) erstrecken, von der ersten Oberfläche der kontinuierlichen oberen Blattbahn (80), und
- Anordnen einer Anordnung von absorbierenden Kernen (26) zwischen der kontinuierlichen oberen Blattbahn (80) und der kontinuierlichen hinteren Blattbahn (70), um eine Rahmenkette (98) zu bilden,
- Schneiden der Rahmenkette (98), um einzelne absorbierende Hygieneartikel (10) zu bilden.

10. Verfahren nach Anspruch 9, wobei der Schritt des Anbringens der beiden kontinuierlichen Beinmanschettenbünde (84) an der ersten Oberfläche der kontinuierlichen oberen Blattbahn (82) ausgeführt wird, bevor die Anordnung von absorbierenden Kernen (26) zwischen der kontinuierlichen oberen Blattbahn (80) und der kontinuierlichen hinteren Blattbahn (70) eingeschoben wird.

11. Verfahren nach Anspruch 10, wobei der Schritt des Anbringens der beiden kontinuierlichen Beinmanschettenbünde (84) an der ersten Oberfläche der kontinuierlichen oberen Blattbahn (80) ausgeführt wird, nachdem die Anordnung von absorbierenden Kernen (26) zwischen der kontinuierlichen oberen Blattbahn (80) und der kontinuierlichen hinteren Blattbahn (70) eingeschoben wird.

12. Verfahren nach einem der Ansprüche 9 bis 11, umfassend ein Bilden von nicht elastischen Abschnitten (44, 46) auf den beiden kontinuierlichen Beinmanschettenbünden (84), die in einer Längsrichtung voneinander beabstandet sind, und ein Überlappen und Befestigen der gegenüberliegenden seitlichen Abschnitte (50) der Dichtungselemente (48) teilweise auf den nicht elastischen Abschnitten (44, 46) und teilweise auf elastischen Abschnitten (52) der kontinuierlichen elastischen Beinmanschettenbünde (84).

## Revendications

1. Article sanitaire absorbant comprenant :
- un châssis (12) présentant un axe longitudinal (X), une section (16, 18) de taille avant et arrière, et une section d'entrejambe (20) intermédiaire entre la section (16, 18) de taille avant et arrière, le châssis (12) incluant une feuille supérieure (22) présentant une surface externe qui, lors de l'utilisation, est en contact avec la peau de l'utilisateur, une feuille arrière (24), et une âme absorbante (26) disposée entre la feuille supérieure (22) et la feuille arrière (24),
- une paire de rabats de jambe (34) élastiques s'étendant au moins en partie le long de directions parallèles audit axe longitudinal (X) et attachés à ladite surface externe de la feuille supérieure (22),
- au moins un élément d'étanchéité (48) présentant un axe longitudinal orienté dans une direction transversale (Y) orthogonale audit axe longitudinal (X), dans lequel le au moins un élément d'étanchéité (48) présente une longueur dans la direction transversale (Y) qui est inférieure à la longueur dans la direction transversale du châssis (12), dans lequel le au moins un élément d'étanchéité (48) est attaché à ladite surface externe de la feuille supérieure (22) dans au moins une parmi ladite section (16, 18) de taille avant et arrière, dans lequel ledit au moins un élément d'étanchéité (48) n'est pas étiré dans ladite direction transversale (Y) et présente deux portions latérales (50) opposées chevauchées et fixées à des portions d'extrémité élastiques (52) desdits rabats de jambe (34) élastiques, une portion externe centrale (54) s'étendant entre lesdites deux portions latérales (50) opposées et fixée à la surface externe de la feuille supérieure (22) et une portion interne centrale (56) s'étendant entre lesdites deux portions latérales (50) opposées et détachée de la surface externe de la feuille supérieure (22), dans lequel ladite portion interne centrale (56) de l'élément d'étanchéité (48) est surélevée à partir de la surface externe de la feuille supérieure (22) et forme une poche ouverte vers ladite section d'entrejambe (20),
**caractérisé en ce que** ledit au moins un élément d'étanchéité (48) est fait d'un matériau non élastique, et **en ce que** lesdites deux portions latérales (50) opposées de l'élément d'étanchéité (48) s'étendent partiellement sur des portions d'extrémité non élastiques (44, 46) et partiellement sur des portions d'extrémité élastiques (52) des rabats de jambe (34) élastiques.

2. Article sanitaire absorbant selon la revendication 1, dans lequel ledit au moins un élément d'étanchéité (48) comprend un matériau élastique non étiré dans ladite direction transversale (Y).

3. Article sanitaire absorbant selon l'une quelconque des revendications précédentes, dans lequel ledit au moins un élément d'étanchéité (48) comprend une couche de matériau plastique imperméable.

4. Article sanitaire absorbant selon la revendication 3, dans lequel ladite couche de matériau plastique imperméable forme la surface de l'élément d'étanchéité (48) face à la surface externe de la feuille supérieure (22).

5. Article sanitaire absorbant selon l'une quelconque des revendications précédentes, dans lequel ledit au moins un élément d'étanchéité comprend au moins une couche de matériau non tissé.

6. Article sanitaire absorbant selon la revendication 5, dans lequel ladite au moins une couche de matériau non tissé forme la surface de l'élément d'étanchéité (48) qui, lors de l'utilisation, est en contact avec la peau de l'utilisateur.

7. Article sanitaire absorbant selon l'une quelconque des revendications précédentes, dans lequel ledit au moins un élément d'étanchéité (48) présente un bord externe aligné sur un bord transversal correspondant de la section (16, 18) de taille avant ou arrière respective.

8. Article sanitaire absorbant selon l'une quelconque des revendications précédentes, dans lequel le châssis (12) présente au moins un bord transversal ayant un profil de forme non droite.

9. Procédé de production d'articles sanitaires absorbants, comprenant :
- le fait de prévoir une toile (80) continue de feuille supérieure,
- le fait d'attacher deux bandes (84) continues de rabats de jambe à une première surface de ladite toile (80) continue de feuille supérieure,
- le fait de prévoir une toile (88) continue d'étanchéité faite d'un matériau non élastique,
- le fait de découper ladite toile (88) continue d'étanchéité dans une direction transversale de façon à former des éléments d'étanchéité (48) individuels allongés dans une direction transversale (Y) et espacés les uns des autres dans une direction longitudinale,
- après l'attache des deux bandes (84) continues de rabats de jambe à la première surface de la toile (80) continue de feuille supérieure, le fait de faire se chevaucher et de fixer des portions latérales (50) opposées des éléments d'étanchéité (48) non étirés dans la direction transversale (Y) à des portions élastiques (52) des deux bandes (84) continues de rabats de jambe, dans lequel lesdites deux portions latérales (50) opposées de l'élément d'étanchéité (48) s'étendent partiellement sur des portions d'extrémité non élastiques (44, 46) et partiellement sur des portions d'extrémité élastiques (52) des rabats de jambe (34) élastiques, le fait de fixer des portions externes centrales (54) des éléments d'étanchéité (48) s'étendant entre des portions latérales (50) opposées respectives à la première surface de la toile (80) continue de feuille supérieure et le fait de laisser des portions internes centrales (56) des éléments d'étanchéité (48) s'étendant entre des portions latérales (50) opposées respectives détachées de la première surface de la toile (80) continue de feuille supérieure, et
- le fait d'agencer un réseau d'âmes absorbantes (26) entre lesdites toile (80) continue de feuille supérieure et toile (70) continue de feuille arrière, de façon à former une chaîne (98) de châssis,
- la découpe de ladite chaîne (98) de châssis de façon à former des articles sanitaires absorbants (10) individuels.

10. Procédé selon la revendication 9, dans lequel l'étape d'attache des deux bandes (84) continues de rabats de jambe à la première surface de la toile (82) continue de feuille supérieure est effectuée avant la prise en sandwich du réseau de coeurs absorbants (26) entre la toile (80) continue de feuille supérieure et la toile (70) continue de feuille arrière.

11. Procédé selon la revendication 10, dans lequel l'étape d'attache des deux bandes (84) continues de rabats de jambe à la première surface de la toile (80) continue de feuille supérieure est effectuée après la prise en sandwich du réseau de coeurs absorbants (26) entre la toile (80) continue de feuille supérieure et la toile (70) continue de feuille arrière.

12. Procédé selon l'une quelconque des revendications 9 à 11, comprenant la formation sur les deux bandes (84) continues de rabats de jambe de portions non élastiques (44, 46) espacées les unes des autres dans une direction longitudinale, et le chevauchement et la fixation desdites portions latérales (50) opposées desdits éléments d'étanchéité (48) partiellement sur lesdites portions non élastiques (44, 46) et partiellement sur des portions élastiques (52) des bandes (84) continues de rabats de jambe élastiques.
